# EUROPEAN PATENT APPLICATION

(11) **EP 3 626 181 A1**
(43) Date of publication of application: **25.03.2020**
(21) Application number: 18196134.3
(22) Date of filing: 23.09.2018
(51) Int. Cl.: A61B 17/064, A61F 2/24

(54) **A CLIP FOR FIXATION OF A MEDICAL IMPLANT TO TISSUE**

(71) Applicant: Medtentia International Ltd Oy, 02600 Espoo (FI)
(72) Inventor: Waldron, Paul, 02600 Espoo (FI); Davis, Matthew, 02600 Espoo (FI)
(74) Representative: Invent Horizon IP

(57) **Abstract**

A clip for fixation of a medical implant to tissue is disclosed, having a delivery shape and a relaxed shape, comprising a proximal cylindrical portion to be arranged in a tubular lumen of a catheter, and first and second resilient legs having respective proximal ends attached to the cylindrical portion, the proximal ends being separated by a width of a bridging section of the cylindrical portion, the proximal ends being connected in an inward direction, extending from each of the proximal ends to the central longitudinal axis of the cylindrical portion, the bridging section together with the first and second legs forming an opening to receive an annuloplasty implant, each of the first and second legs having a distal portion to pierce said tissue, and wherein in said relaxed shape each of the distal portions are curved to deflect from the central longitudinal axis. A related method is disclosed.

## Description

### Field of the Invention

This invention pertains in general to the field of clips being attachable to tissue. More particularly the invention relates to clips for fixating annuloplasty implants to heart valve tissue, and a method therefore.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure. The annuloplasty ring is typically implanted around the annulus of the heart valve.

It is a challenge to achieve correct positioning of annuloplasty implants at the heart valve and fixate the implant in the correct position. Suturing devices for annuloplasty implants have disadvantages that makes it difficult to suture in the correct position, thereby resulting insufficient suturing strength, and also in a very time-consuming procedure, which increases the risks for the patient. Furthermore, suturing devices are often not sufficiently compact for catheter based procedures.

A problem with prior art fixation systems and fixation devices, such as clips, is also the complexity of the devices which requires a several operating steps in which the several movable parts must be engaged in sequence. The procedure thus becomes more complicated and time consuming. Frequently the target site may be of complex anatomy and there may be movement, such as the motion of the beating heart in addition to the operator's movements, that adds to the challenge of positioning a clip and fixating the implant effectively. A problem with prior art clips is limited securing strength and difficulties in attaching the clips at the desired site with high accuracy. A further problem in the prior art is thus also to achieve a reliable fixation at the annulus of the heart valve. An annuloplasty implant is intended to function for years and years, so it is critical with long term stability in this regard.

The above problems may have dire consequences for the patient and the health care system. Patient risk is increased.

Hence, an improved clip for fixation of a medical implant to tissue would be advantageous and in particular allowing for avoiding more of the above mentioned problems and compromises, and in particular ensuring secure fixation of the annuloplasty implant, during the implantation phase, and for long-term functioning, in addition to a less complex procedure, and increased patient safety. A related method would also be advantageous.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect a clip for fixation of a medical implant to tissue is provided. The clip has a delivery shape and a relaxed shape and comprises a proximal cylindrical portion to be arranged in a tubular lumen of a catheter and being movable in a longitudinal direction thereof when arranged therein, whereby the cylindrical portion extend along a central longitudinal axis being parallel with the longitudinal direction when arranged in the tubular lumen. The clip comprises first and second resilient legs, the first and second legs having respective proximal ends attached to the cylindrical portion, the proximal ends being separated by a width of a bridging section of the cylindrical portion, whereby the proximal ends are connected in an inward direction, extending from each of the proximal ends to the central longitudinal axis of the cylindrical portion, the bridging section together with the first and second legs forming an opening to receive an annuloplasty implant, each of the first and second legs having a distal portion to pierce said tissue, and wherein in said relaxed shape each of the distal portions are curved to deflect from the central longitudinal axis.

According to a second aspect a system is provided comprising a clip according to the first aspect and a catheter, wherein the first and second legs are resiliently deformable to the delivery shape when a compression force is applied by a distal part of the catheter on respective distal tips of the distal portions in the inward direction, wherein, in use, when the compression force is removed, the distal portions spring towards the relaxed shape to apply a force onto the distal part having a force vector component in the longitudinal direction towards the cylindrical portion to pull the cylindrical portion in the longitudinal direction towards the distal part.

According to a third aspect a method of delivering a clip to a target site is provided. The clip comprising first and second resilient legs having respective proximal ends attached to a proximal cylindrical portion, the proximal ends being separated by a width of a bridging section of the cylindrical portion, whereby the proximal ends are connected in an inward direction, extending from each of the proximal ends to a central longitudinal axis of the cylindrical portion, each of the first and second legs having a distal portion to pierce tissue at the target site, wherein in a relaxed shape of the clip each of the distal portions are curved to deflect from the central longitudinal axis. The method comprises arranging a distal cylindrical portion of the clip in a tubular lumen extending in a longitudinal direction of a catheter, compressing the first and second legs by applying a compression force on respective distal tips of the distal portions, delivering the catheter to the target site at which an annuloplasty ring is positioned, pushing the cylindrical portion a distance along the longitudinal direction towards a distal part of the catheter so that the compression force is removed, whereby the distal portions spring towards the relaxed shape to apply a force onto the distal part having a force vector component in the longitudinal direction towards the cylindrical portion to pull the cylindrical portion in the longitudinal direction towards the distal part, and whereby the distal portions pierce into the tissue at either side of said annuloplasty ring to fixate the position of the annuloplasty ring at the target site.

Further examples of the invention are defined in the dependent claims, wherein features for the second and subsequent aspects are as for the first aspect mutatis mutandis.

Some examples of the disclosure provide for a facilitated positioning of an annuloplasty implant at a heart valve.

Some examples of the disclosure provide for a facilitated fixation of an annuloplasty implant at a heart valve.

Some examples of the disclosure provide for a less time-consuming fixation of an annuloplasty to a target site.

Some examples of the disclosure provide for securing long-term functioning and position of an annuloplasty implant.

Some examples of the disclosure provide for a reduced risk of damaging the anatomy of the heart such as the annulus or the valve leaflets.

Some examples of the disclosure provide for a more secure implantation of an annuloplasty implant in narrow anatomies.

Some embodiments of the invention provide for secure fixation of an annuloplasty implant on either side of a heart valve.

Some embodiments of the invention provide for increased accuracy when attaching a clip to a beating heart.

Some embodiments of the invention provide for a more secure and stronger attachment with a clip and improved fixation of annuloplasty rings at a heart valve.

Some embodiments of the invention provide for reducing the risk of damaging tissue with a clip.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 a is a schematic illustration of a clip in a side view;
Fig. 1b is a schematic illustration of a clip in a top-down view;
Fig. 1c is a schematic illustration of a clip in a side view, along axis A in Fig. 1a;
Figs. 2a-f are schematic illustrations of a clip being delivered from a catheter;
Fig. 3a is a schematic illustration of a clip fixing an annuloplasty implant to tissue;
Fig. 3b is a schematic illustration of a plurality of clips fixing an annuloplasty implant to tissue at a heart valve;
Fig. 4a is a schematic illustration of a clip in a side view, where the legs of the clip are in a straight configuration before heat setting in a curved shape;
Fig. 4b is a schematic illustration of a clip in a side view, along axis A in Fig. 4a, where the legs of the clip are in a straight configuration before heat setting in a curved shape;
Fig. 4c is a schematic illustration of the clip of Fig. 4b in a top-down view;
Figs. 5a-b are schematic illustration of a clip in a side view and in a top-down view, respectively, when attached to tissue;
Figs. 6a-b are schematic illustration of a clip in a side view and in a top-down view, respectively, when attached to tissue;
Figs. 7a-b are schematic illustration of a clip in a side view and in a top-down view, respectively, when attached to tissue;
Figs. 8a-b are schematic illustration of a clip in a side view and in a top-down view, respectively, when attached to tissue;
Fig. 9 is a schematic illustration of a clip in a side view;
Fig. 10 is a schematic illustration of a clip in a top-down view;
Fig. 11 is a schematic illustration of a clip in a side view when arranged in a catheter;
Fig. 12a is a flow chart of a method of delivering a clip to a target site; and
Fig. 12b is another flow chart of a method of delivering a clip to a target site.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

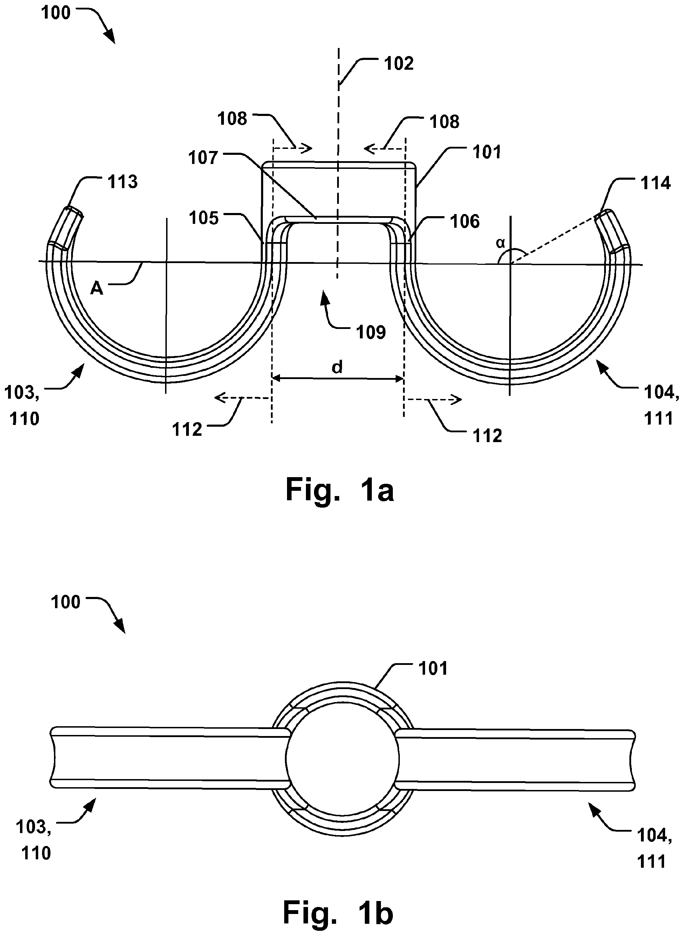

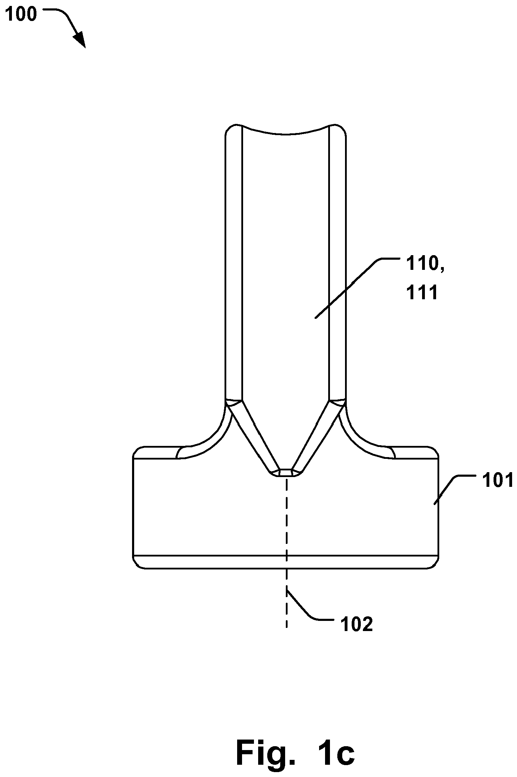

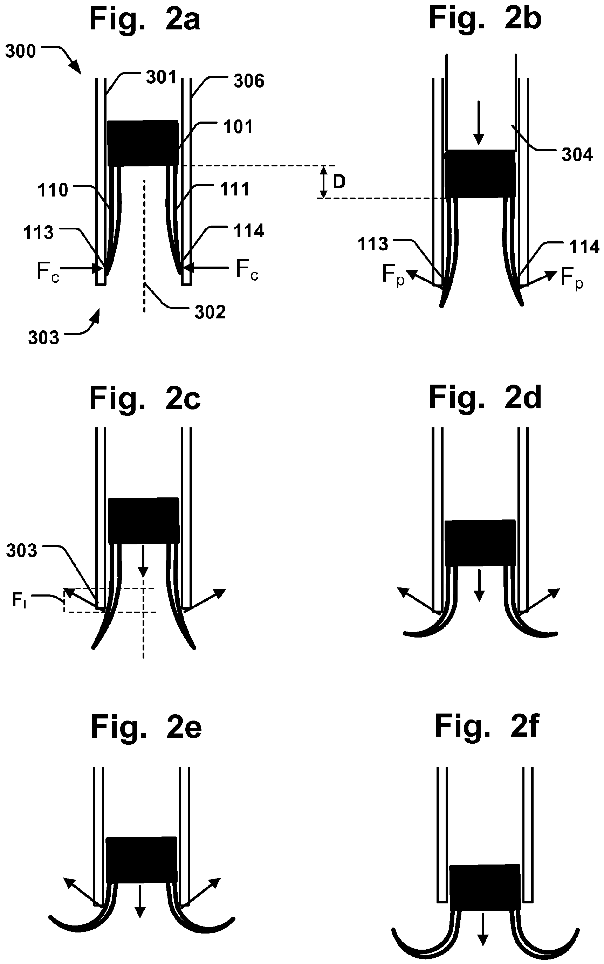

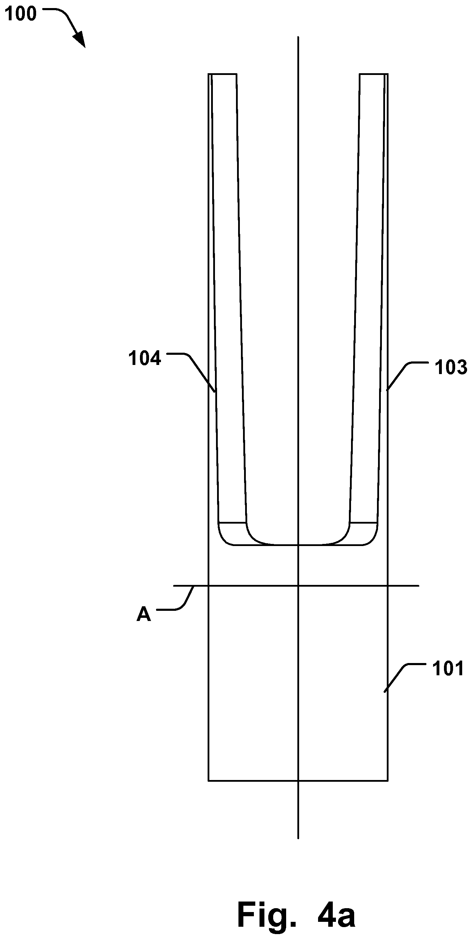

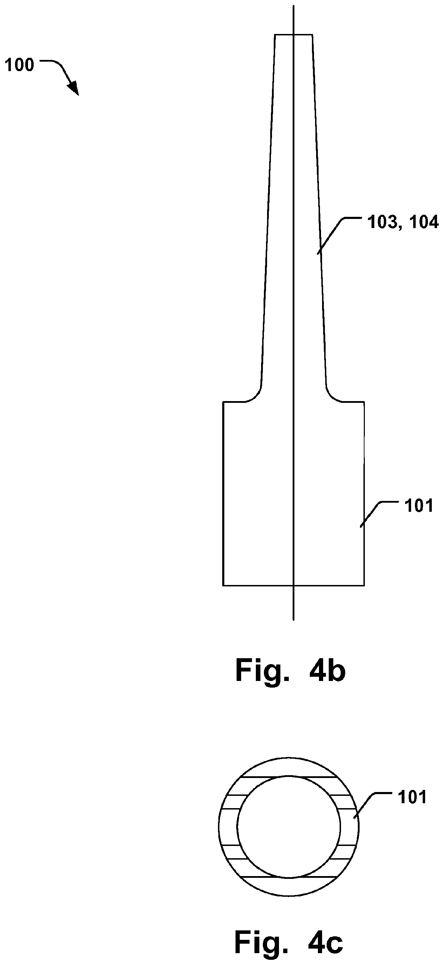

Figs. 1 - 11 are schematic illustrations of a clip 100 for fixation of a medical implant to tissue. The clip 100 has a delivery shape and a relaxed shape. An example of a delivery shape is shown in Fig. 2a where the clip 100 is restrained in a catheter 306 for delivery to a target site. Fig. 2f show an example of a relaxed shape of the clip 100, when the forces acting on the clip 100 from the catheter 306 have been removed. The shape of the clip 100 in the relaxed shape may be set in a heat treatment procedure. In one example, the clip 100 may be cut from a tubular piece of material, as schematically illustrated in Figs. 4a-c. I.e. legs 104, 105, of the clip 100 may be formed by cutting out the intermediate portions of such tubular piece of material. The legs 104, 195, may then be bent into the desired shape, e.g. as schematically illustrated in the example of Figs. 1a-c, and the clip 100 may undergo a heat treatment procedure for setting the desired shape, to obtain the aforementioned relaxed shape of the clip 100, which corresponds to the shape seen in e.g. Figs. 1a-c.

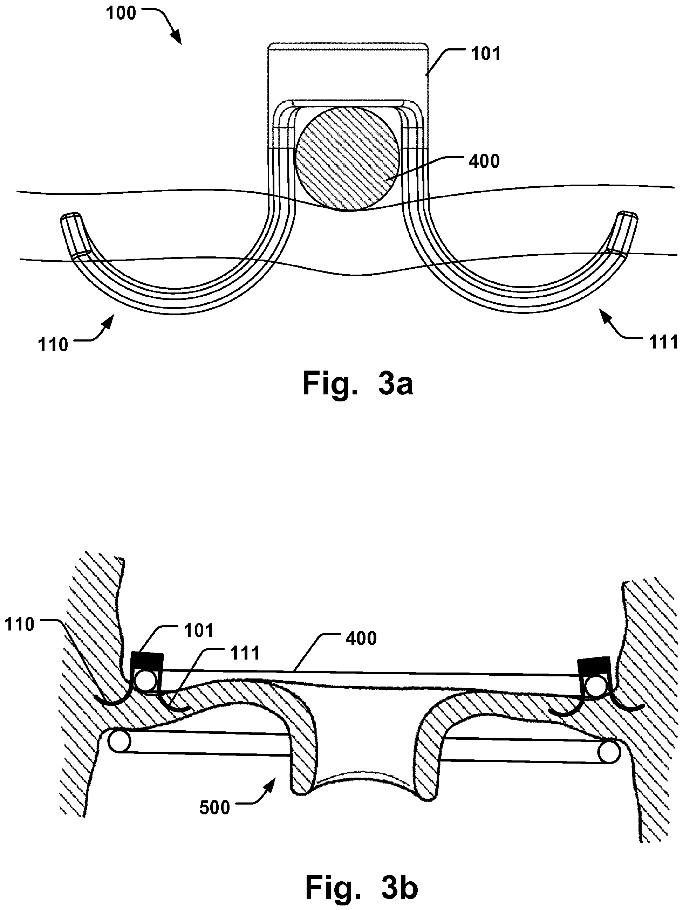

The clip 100 comprises a proximal cylindrical portion 101 to be arranged in a tubular lumen 301 of a catheter 306, as illustrated in e.g. Fig. 2a. The proximal cylindrical portion 101 is movable in a longitudinal direction 302 of the catheter 306 when arranged therein. The cylindrical portion 101 has a central longitudinal axis 102, along which the cylindrical portion 101 extends. The central longitudinal axis 102 is thus parallel with the longitudinal direction 302 when arranged in the tubular lumen 301. The clip 100 comprises first and second resilient legs 103, 104, having respective proximal ends 105, 106, attached to the cylindrical portion 101, as illustrated in the example of Fig. 1a. The proximal ends 105, 106, are separated by a width (d) of a bridging section 107 of the cylindrical portion 101. The proximal ends 105, 106, are thus connected in an inward direction 108, extending from each of the proximal ends 105, 106, to the central longitudinal axis 102 of the cylindrical portion 101. The bridging section 107 together with the first and second legs 103, 104, form an opening 109 to receive an annuloplasty implant 400, as schematically illustrated in Fig. 3a in conjunction with Fig. 1a. Each of the first and second legs 103, 104, has a distal portion 110, 111, configured to pierce tissue at the heart valve, as schematically illustrated in Figs. 3a-b. In the relaxed shape of the clip 100, each of the distal portions 110, 111, are curved to deflect from the central longitudinal axis 102. As mentioned Fig. 1a show an example where the clip 100 is in the relaxed shape, and the distal portions are curved away in a non-parallel orientation with respect to the longitudinal axis 102. As the clip 100 is released from the constraint from the catheter 306, the first and second legs 103, 104, and the associated distal portions 110, 111, will progressively move from a straightened shape, as exemplified in Fig. 2a, towards the relaxed shape, as seen in e.g. Fig. 2f. The motion of the clip 100 between the aforementioned shapes will be driven by the potential energy stored in the deformed material of the clip 100 when confined in the catheter 306 (Fig. 2a). The potential energy is accumulated when a force is applied onto the clip 100 to push it inside the catheter 306. As will be described in further detail below, since the distal portions 110, 111, are curved from the longitudinal axis 102 in the relaxed shape, the clip 100 will drive or propel itself out from the catheter 306 due to the curved distal portions 110, 111, gradually applying a force onto the distal part 303 of the catheter 306 as illustrated in the sequence of motion in Figs. 2b-f. Having a clip 100 shaped as specified above thus allows for ejecting the clip 100 from the catheter 306 by the potential energy stored within the material of the clip 100. A small initial push with e.g. a wire 304, as shown in Fig. 2b, is thus sufficient for the clip 100 to propel itself from the catheter 306 towards the tissue and pierce the tissue. In some examples it is not necessary to have a separate element for pushing the clip 100 as seen in Fig. 2b, but instead the catheter 306 can be tapped onto the tissue which provides for the required amount of inertia transferred to the clip 100 so that it moves into the position of Fig. 2b. Then, as explained above, the striving force towards the relaxed curved shape will drive the clip 100 forwards as shown in Figs. 2b-f. The forward force of the clip 100 will cause the distal portions 110, 111, to pierce into the tissue as shown in Figs. 3a-b.

Hence, having curved distal portions 110, 111, as specified provides for facilitating the fixation of the clip 100 into the tissue, without having to actively force or push the clip 100 forward into the tissue. The fixation is thus simplified and can be completed in less amount of time. Furthermore, by having a proximal cylindrical portion 101 the stability of the clip 100 is improved when arranged in a catheter 306, since the cylindrical portion 101 will slide inside the tubular lumen 301 and prevent undesired tilting or tumbling of the clip 100 while being delivered and ejected from the catheter 306. A robust clip 100 is thus provided, which can be delivered with greater certainty of correct positioning. Accuracy is thus increased and the risk of dislocated clips, and thereby insufficient fixation of an implant, can thus be minimized. Furthermore, having a bridging section 107 together with the first and second legs 103, 104, forming an opening 109 to receive an annuloplasty implant 400, increases the stability of the fixation of the implant 400. The opening 109 can be dimensioned to fit various implants 400, which will prevent excessive relative movement between the clip 100 and the implant 400.

In some examples the distal portions 110, 111, are curved so respective distal tips 113, 114, thereof are oriented back towards the proximal cylindrical portion 101, as illustrated in the example of Fig. 1a and 3a. This allows for driving the distal portions 110, 111, into the tissue from the opposite side relative to the tissue side which is engaged by the implant 400, as seen in Fig. 3a. This prevents damages to surrounding tissue by the distal tips 113, 114, since these are embedded into the tissue on the opposite side, as seen in Fig. 3a. The clip 100 in Fig. 3a, i.e. when embedded into the tissue, may be referred to as being in its relaxed shape since it has been released from the constraint by the catheter 306, although it should be understood that the tissue may provide a counter force onto the clip 100. The shape of the clip 100 in the implanted state is however referred to as the relaxed shape in the present disclosure to simplify the discussion.

As seen in Fig. 3b, a plurality of clips 100 may be placed around the annuloplasty implant 400 to achieve a secure fixation thereof. Although the annuloplasty implant 400 is shown as a helix-shaped implant inserted through the valve with first and second rings on opposite sides of the valve in the example of Fig. 3b, it should be understood that the clip 100 may fixate single ring annuloplasty implants with the same advantageous benefits as described above. The implant 400 may comprise a shape-memory material, so that the first and second rings assume a coiled configuration after having been ejected from a delivery catheter (not shown). While positioned in the delivery catheter the implant 400 may be stretched in an elongated shape. Alternatively, the implant 400 may be arranged in the coiled configuration when being delivered to the target site, in which case it may be implanted at the target site for example by incision between the ribs or by opening the chest. The present disclosure, and the associated advantages described for the various examples of the clip 100 applies to both such variants of the implant 400.

Turning again to Figs. 2a-f, the first and second legs 103, 104, may be resiliently deformable from the relaxed shape to the delivery shape in which each of the distal portions extend substantially along the longitudinal axis 302 (Fig. 2a). The first and second legs 103, 104, may thus be resiliently deformable to the delivery shape when a compression force (F_{c}) is applied on respective distal tips 113, 114, of the distal portions 110, 111. The compression force F_{c} may be applied by the distal part 303 of the catheter 306 as exemplified in Fig. 2a. When the compression force F_{c} is removed, either by pushing slightly by a wire 304 (Fig. 2b) or by tapping the catheter 306 against the tissue or applying an otherwise oscillating motion onto the catheter 306, so that the distal tips 113, 114, are released from the catheter 306 (Fig. 2b), the distal portions 110, 111, spring towards the relaxed shape and pulls the cylindrical portion 101 in the longitudinal direction 302 as shown in the sequence of Figs. 2b-f. As elucidated above, as the distal portions 110, 111, progressively move to assume their fully curved shapes in the relaxed state, the distal portions 110, 111, will grasp around the edges of the distal part 303, and thereby push with a force (Fₚ) against the distal part 303 which pulls the entire clip 100 in the opposite direction towards the opening of the catheter 306.

In the relaxed shape, each of the distal portions 110, 111, may be curved in an outward direction 112, substantially opposite the inward direction 108, as shown in the examples of Figs. 1a-c, 2a-f, 3a, 5a-b, 9. This provides for an effective anchoring of the clip 100 into the tissue and a secure fixation of an annuloplasty implant 400.

Thus, the first and second legs 103, 104, may be resiliently deformable in the inward direction 108 from the relaxed shape to the delivery shape when a compression force (F_{c}) is applied on respective distal tips 113, 114, of the distal portions in the inward direction 108, as described above and shown in Fig. 2a.

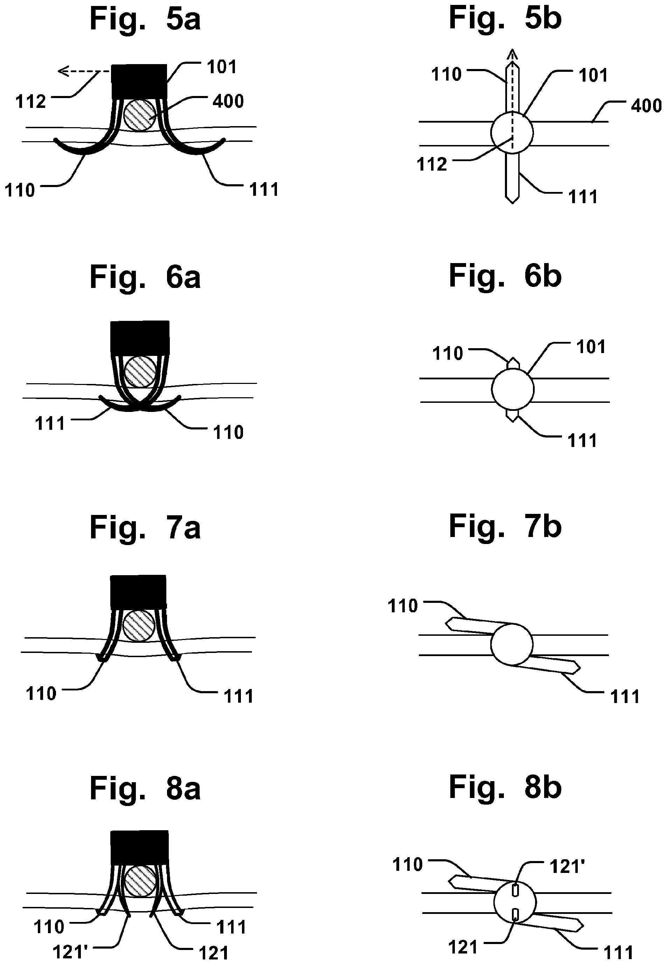

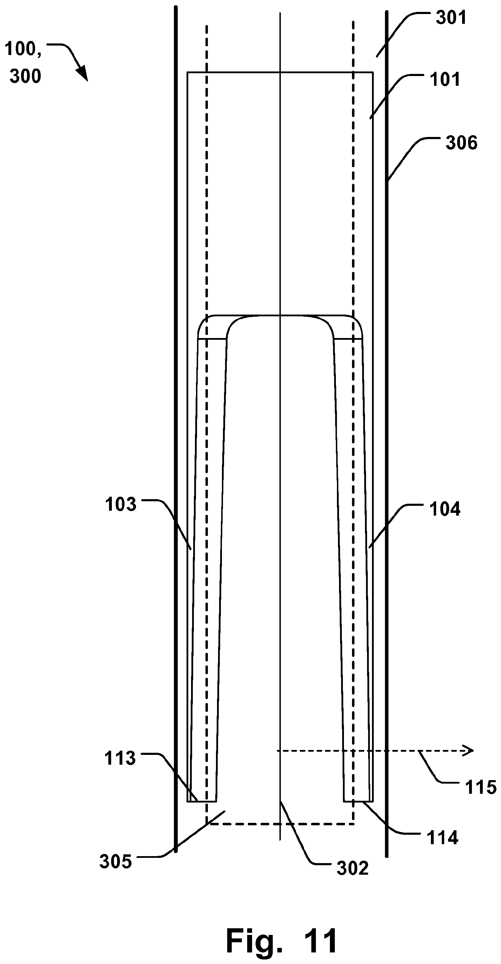

In another example, when the clip 100 is in its relaxed shape, each of the distal portions 110, 111, may be curved in the inward direction 108, as schematically shown in Figs. 6a-b. This provides for reducing how far the distal portions 110, 111, extend from the cylindrical portion 101, which may be advantageous in some situations and anatomies. As seen in the top-down view of Fig. 6b, the extended distance of distal portions 110, 111, is reduced since the distal portions 110, 111, are curved in the inward direction 108 (Fig. 6a, in conjunction with Fig. 1a) to wrap at least partly around the implant 400. As explained further below in relation to Fig. 11, a guide element 305 may apply a force onto the legs 103, 104, for separation thereof in a catheter 306.

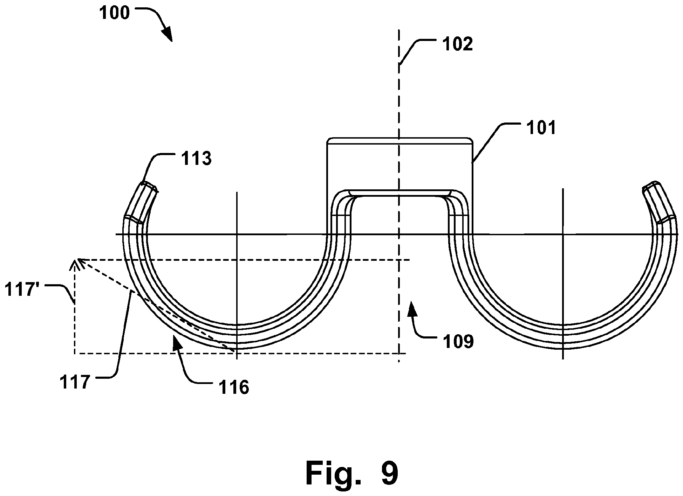

Each of the distal portions 110, 111, may be curved so that at least a section 116 thereof extends in a direction 117 having a vector component 117' parallel with the central longitudinal axis 102 and extending along the central longitudinal axis 102 from the opening 109 towards the cylindrical portion 101. Figs. 2f and Fig. 9 show such examples where at least a section 106 of the distal portions 110, 111, are curved in the direction towards the cylindrical portion 101. I.e. the section 106 is angled so that the extension of the curved shape has a component 117' extending in a direction towards the cylindrical portion 101. The distal portions 110, 11, may thus engage the tissue from the opposite side of the cylindrical portion 101 as shown in Fig. 3a, providing for increasing the fixation strength while avoiding having distal tips 113, 114, extending into the surrounding space.

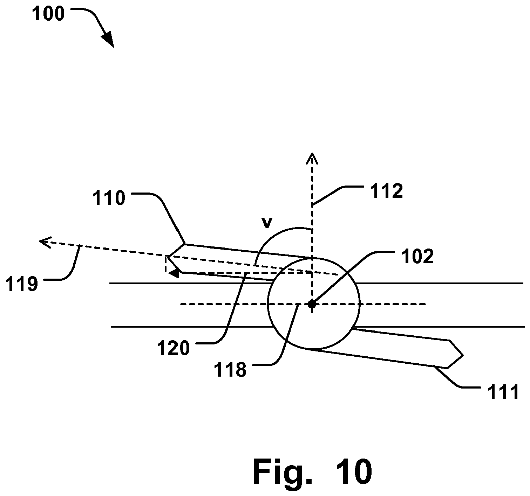

The clip 100 has a transverse direction 118 which is orthogonal to the outward direction 112 and the central longitudinal axis 102, as schematically illustrated in Fig. 10. In one example, in the relaxed shape of the clip 100, each of the distal portions 110, 111, may be curved along the transverse direction 118. Each of the distal portions 110, 111, may thus extend along a direction 119 having a vector component 120 along the transverse direction 118, as further shown in the example of Fig. 10. The angle (v) may be optimized depending on the application and anatomy. In some examples the angle (v) may be close to 90 degrees, when it is desired to have the legs 103, 104, parallel with the implant 400. In some examples the angle may be in the range 45-90 degrees. Figs. 7a-b are also schematic illustrations showing the distal portions 110, 111, being curved towards the transverse direction 118. As with the example in Figs. 6a-b, this provides for reducing the length by which the distal portions 110, 111, extend perpendicular to the annuloplasty implant 400. This may be advantageous in some situations where it is desired to reduce the distance pierced at the annulus, perpendicular to the implant 400. Further, in some situations when the implant 400 and clip 100 is placed closer to the edges of the leaflets, it may be desirable to reduce the extension of the leg 103, 104, facing the leaflet.

The distal portions 110, 111, may be curved in opposite directions along the transverse direction 118, as shown in the examples of Figs. 7a-b and 10. This provides for attaining a high fixation strength while reducing the length extended by the legs 103, 104, perpendicular to the direction of the implant 400, i.e. in the outward direction 112.

The clip 100 may comprise at least one secondary leg 121, 121', attached to the cylindrical portion 101. The at least one secondary leg 121, 121', extends along the central longitudinal axis 102 and is curved in the inward direction 108, as schematically illustrated in Figs. 8a-b in conjunction with Fig. 1a. Thus, the clip 100 may comprise legs 103, 104, having distal portions 110, 111, curved in the transverse direction 118 as explained above, as well as at least one secondary leg 121, 121', curved in the inward direction 108 as shown in the example of Figs. 8a-b. The at least one secondary leg 121, 121', provides for further stabilizing the clip 100 in relation to the implant 400 due to the additional force that may be exerted from the at least one secondary leg 121, 121', onto the implant 400. Resistance to rotational movement of the clip 100 may thus be increased due to the added support from the at least one secondary leg 121, 121', and dislocation from the desired position of the clip 100 can be prevented. This added resistance to rotation may be particularly advantageous when the legs 103, 104, are curved in the transverse direction 118. In the mentioned example of Figs. 8a-b the clip 100 comprises two secondary leg 121, 121', one on each side of the implant 400, which may provide for a further added support and resistance to rotation or dislocation.

In one example the legs 103, 104, are curved in a circular shape, as schematically illustrated in e.g. Fig. 1a. The radius of curvature may be substantially constant in some examples. The angle (α) in Fig. 1a may be in the range 150-180 degrees in some examples. In one example the angle (α) may be 150 degrees, which may provide for a particularly advantageous fixation.

A system 300 is provided comprising a clip 100 as described above in relation to Figs. 1 - 11 and a catheter 306. Turning again to Figs. 2a-f, the first and second legs 103, 104, may be resiliently deformable to the delivery shape when a compression force (F_{c}) is applied by a distal part 303 of the catheter 306 on respective distal tips 113, 114, of the distal portions 110, 111, in the inward direction 108. Hence, in use, when the compression force (F_{c}) is removed, the distal portions 110, 111, spring towards the relaxed shape to apply a force (Fₚ) onto the distal part 303 having a force vector component (Fₗ) in the longitudinal direction 302 towards the cylindrical portion 101 to pull the cylindrical portion 101 in the longitudinal direction 302 towards the distal part 303. Fig. 2c illustrates an example of a direction of such vector component (Fₗ) that act against the distal part 303 to pull the clip 100 out from the catheter 306 by its own motion. The motion is sourced from the release of potential energy stored in the deformed delivery state of the clip 100.

The system 300 may comprise a wire 304 being movable in the tubular lumen 301, as seen in the example of Fig. 2b. The wire 304 is movable to remove the compression force F_{c}, applied by the catheter 306 in the inward direction 108, by pushing the cylindrical portion a distance (D) along the longitudinal direction 302 towards the distal part 303 (Figs. 2a-b).

The system 300 may comprise an annuloplasty implant 400 comprising at least one annuloplasty ring. The opening 109 may be configured to receive the at least one annuloplasty ring and fixate the position of the annuloplasty implant 400 to a heart valve 500, as exemplified in Fig. 3b.

The width (d) of the bridging section 107 of the cylindrical portion 101 may correspond substantially to a diameter of the annuloplasty ring 400, as shown in the example of Fig. 3a in conjunction with Fig. 1a. This provides for a stable fixation of the implant 400 to the clip 100 and tissue.

The system 300 may comprise a guide element 305 in the tubular lumen 301, as schematically illustrated in the example of Fig. 11. The guide element 305 is positionable to separate the first and second legs 103, 104, in a radial direction 115, perpendicular to the longitudinal direction 302 when the clip 100 is arranged in the catheter 306. This provides for separating the legs 103, 104, in a straight configuration in case the distal portions 110, 111, have a relaxed shape which curves in the inward direction 108 as shown in Figs. 6a-b. The distal portions 110, 111, may accordingly be separated while engaging the implant 400 so that when the guide element is 305 withdrawn, the distal portions 110, 111, are able to move forward into position on either side of the implant 400 as seen in Figs. 6a-b. Thus, in the example of Fig. 11, the guide element 305 applies a force onto the distal portions 113, 114, and when the force is removed, e.g. by retracting the guide element 305 slightly, the distal portions 110, 111, will spring towards the curved relaxed shape and exert a force onto the guide element 305 corresponding to (Fₚ) in the example of Figs. 2b-f. In a similar manner as described in relation to Figs. 2a-f, the clip 100 will propel or drive itself forward out from the catheter 306 once the guide element 305 as released the force on the distal portions 113, 114.

The guide element 305 may be positionable to extend through the proximal cylindrical portion 101 of the clip 100. The cylindrical portion 101 may thus have a through opening, which may be provided by forming the clip 100 from a tubular material. The guide element 305 may thus be conveniently advanced through the clip 100 to separate the first and second legs 103, 104, as described above.

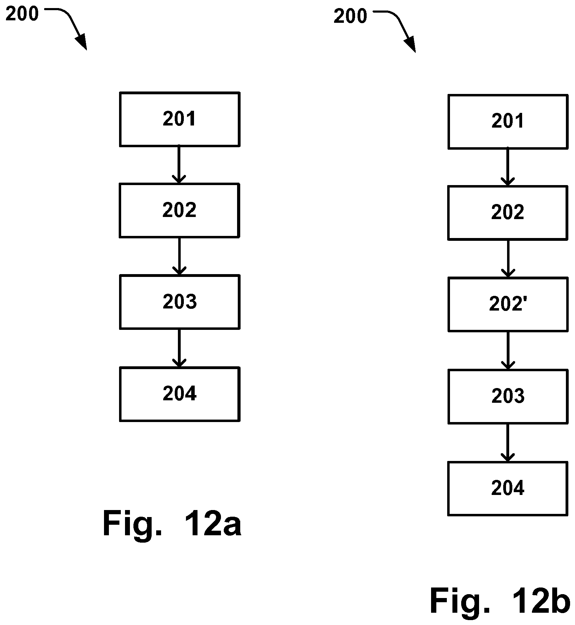

A method 200 of delivering a clip 100 to a target site is provided. The method 200 is schematically illustrated in Fig. 12a, in conjunction with Figs. 1 - 11. The order in which the steps are described should not be construed as limiting, and it is conceivable that the order of the steps may be varied depending on the particular procedure.

As mentioned, the clip 100 comprises first and second resilient legs 103, 104, having respective proximal ends 105, 106, attached to a proximal cylindrical portion 101. The proximal ends 105, 106, are separated by a width (d) of a bridging section 107 of the cylindrical portion 101. The proximal ends 105, 106, are connected in an inward direction 108, extending from each of the proximal ends to a central longitudinal axis 102 of the cylindrical portion 101. Each of the first and second legs 103, 104, has a distal portion 110, 111, to pierce tissue at the target site. In a relaxed shape of the clip 100 each of the distal portions 110, 111, are curved to deflect from the central longitudinal axis.

The method 200 comprises arranging 201 a distal cylindrical portion 101 of the clip 100 in a tubular lumen 301 extending in a longitudinal direction 302 of a catheter 306. The method 200 comprises compressing 202 the first and second legs 103, 104, by applying a compression force (F_{c}) on respective distal tips 113, 114, of the distal portions 110, 111. The method 200 comprises delivering 203 the catheter to the target site at which an annuloplasty ring 400 is positioned. The method 200 comprises pushing 204 the cylindrical portion 101 a distance (D) along the longitudinal direction 302 towards a distal part 303 of the catheter 306 so that the compression force (F_{c}) is removed. As a result, the distal portions 110, 111, spring towards the relaxed shape to apply a force (Fₚ) onto the distal part 303 having a force vector component (Fₗ) in the longitudinal direction 302 towards the cylindrical portion to pull the cylindrical portion 101 in the longitudinal direction 302 towards the distal part 303. The distal portions 110, 111, thereby pierce into the tissue at either side of the annuloplasty ring 400 to fixate the position of the annuloplasty ring 400 at the target site.

The method 200 thus provides for the advantageous benefits as described above in relation to the clip 100 and Figs. 1 - 11.

Fig. 12b is another flowchart of a method 200 of delivering a clip 100 to a target site. In one example, in a relaxed shape of the clip 100 each of the distal portions 110, 111, are curved in an outward direction 112, substantially opposite the inward direction 108. The method 200 may thus comprise compressing 202' the first and second legs 103, 104, in the inward direction 108 by applying a compression force (F_{c}) by the distal part 303 of the catheter 306 on respective distal tips 113, 114, of the distal portions 110, 111. Subsequently removing the compression force (F_{c}), by advancing the clip 100, will cause the clip 100 to drive itself forward as described above by pushing against the distal portion 303.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A clip (100) for fixation of a medical implant to tissue, said clip having a delivery shape and a relaxed shape, the clip comprising;
a proximal cylindrical portion (101) to be arranged in a tubular lumen (301) of a catheter (300) and being movable in a longitudinal direction (302) thereof when arranged therein, whereby the cylindrical portion extend along a central longitudinal axis (102) being parallel with the longitudinal direction when arranged in the tubular lumen,
first and second resilient legs (103, 104),
the first and second legs having respective proximal ends (105, 106) attached to the cylindrical portion,
the proximal ends being separated by a width (d) of a bridging section (107) of the cylindrical portion, whereby the proximal ends are connected in an inward direction (108), extending from each of the proximal ends to the central longitudinal axis (102) of the cylindrical portion,
the bridging section together with the first and second legs forming an opening (109) to receive an annuloplasty implant (400),
each of the first and second legs having a distal portion (110, 111) to pierce said tissue, and
wherein in said relaxed shape each of the distal portions are curved to deflect from the central longitudinal axis.

2. A clip according to claim 1, wherein the first and second legs are resiliently deformable from the relaxed shape to the delivery shape in which each of the distal portions extend substantially along the longitudinal axis,
whereby the first and second legs are resiliently deformable to the delivery shape when a compression force (F_{c}) is applied on respective distal tips (113, 114) of the distal portions, and
wherein, when the compression force is removed, the distal portions spring towards the relaxed shape and pulls the cylindrical portion in the longitudinal direction when arranged in the catheter.

3. A clip according to claim 1 or 2, wherein in said relaxed shape each of the distal portions are curved in an outward direction (112), substantially opposite said inward direction.

4. A clip according to claim 3, wherein the first and second legs are resiliently deformable in the inward direction from the relaxed shape to the delivery shape when a compression force (F_{c}) is applied on respective distal tips (113, 114) of the distal portions in the inward direction.

5. A clip according to claim 1 or 2, wherein in said relaxed shape each of the distal portions are curved in the inward direction.

6. A clip according to any of claims 1 - 5, wherein each of the distal portions are curved so that at least a section (116) thereof extends in a direction (117) having a vector component (117') parallel with the central longitudinal axis and extending along the central longitudinal axis from the opening (109) towards the cylindrical portion.

7. A clip according to any of claims 1 - 5, wherein a transverse direction (118) of the clip is orthogonal to the outward direction (112) and the central longitudinal axis (102),
wherein in said relaxed shape each of the distal portions are curved along the transverse direction, whereby each of the distal portions extend along a direction (119) having a vector component (120) along the transverse direction (118).

8. A clip according to claim 7, wherein the distal portions are curved in opposite directions along the transverse direction.

9. A clip according to claim 7 or 8, comprising at least one secondary leg (121, 121') attached to the cylindrical portion and extending along the central longitudinal axis, wherein the at least one secondary leg is curved in the inward direction.

10. A system (200) comprising a clip according to any of claims 1 - 9 and a catheter (300), wherein the first and second legs are resiliently deformable to the delivery shape when a compression force (F_{c}) is applied by a distal part (303) of the catheter on respective distal tips (113, 114) of the distal portions in the inward direction,
wherein, in use, when the compression force is removed, the distal portions spring towards the relaxed shape to apply a force (Fₚ) onto the distal part (303) having a force vector component (Fₗ) in the longitudinal direction (302) towards the cylindrical portion to pull the cylindrical portion in the longitudinal direction towards the distal part (303).

11. A system according to claim 10, comprising a wire (304) being movable in the tubular lumen, wherein the wire is movable to remove said compression force, applied by the catheter in the inward direction, by pushing the cylindrical portion a distance (D) along the longitudinal direction towards the distal part.

12. A system according to claim 10 or 11, comprising said annuloplasty implant (400) comprising at least one annuloplasty ring, wherein said opening (109) is configured to receive the at least one annuloplasty ring and fixate the position of the annuloplasty implant to a heart valve (500).

13. A system according to any of claims 10- 12, wherein said width (d) corresponds substantially to a diameter of the annuloplasty ring.

14. A system according to any of claims 10- 13, comprising a guide element (305) in the tubular lumen, wherein the guide element is positionable to separate the first and second legs in a radial direction (115), perpendicular to the longitudinal direction, when the clip is arranged in the catheter.

15. A system according to claim 14, wherein the guide element is positionable to extend through the proximal cylindrical portion of the clip.

16. A method (200) of delivering a clip (100) to a target site, the clip comprising first and second resilient legs (103, 104) having respective proximal ends (105, 106) attached to a proximal cylindrical portion (101), the proximal ends being separated by a width (d) of a bridging section (107) of the cylindrical portion, whereby the proximal ends are connected in an inward direction (108), extending from each of the proximal ends to a central longitudinal axis (102) of the cylindrical portion, each of the first and second legs having a distal portion (110, 111) to pierce tissue at the target site, wherein in a relaxed shape of the clip each of the distal portions are curved to deflect from the central longitudinal axis, the method comprising
arranging (201) a distal cylindrical portion (101) of the clip in a tubular lumen (301) extending in a longitudinal direction (302) of a catheter (306),
compressing (202) the first and second legs by applying a compression force (F_{c}) on respective distal tips (113, 114) of the distal portions (110, 111),
delivering (203) the catheter to the target site at which an annuloplasty ring (400) is positioned,
pushing (204) the cylindrical portion a distance (D) along the longitudinal direction towards a distal part (303) of the catheter so that the compression force is removed, whereby the distal portions spring towards the relaxed shape to apply a force (Fₚ) onto the distal part (303) having a force vector component (Fₗ) in the longitudinal direction (302) towards the cylindrical portion to pull the cylindrical portion in the longitudinal direction towards the distal part (303), and whereby the distal portions pierce into the tissue at either side of said annuloplasty ring to fixate the position of the annuloplasty ring at the target site.

17. Method according to claim 16, wherein, in the relaxed shape of the clip 100 each of the distal portions 110, 111, are curved in the outward direction 112, substantially opposite the inward direction 108, the method comprising
compressing (202') the first and second legs in the inward direction by applying a compression force (F_{c}) by the distal part (303) of the catheter on respective distal tips (113, 114) of the distal portions (110, 111)
